# FASCICULE DE BREVET EUROPEEN

(11) **EP 3 277 233 B1**
(45) Date de publication et mention de la délivrance du brevet: **03.07.2024**
(21) Numéro de dépôt: 15717226.3
(22) Date de dépôt: 31.03.2015
(51) Int. Cl.: A61F 9/00, B65D 47/18

(54) **DISPOSITIF DE DISTRIBUTION DE LIQUIDE HORS D'UN FLACON DE CONDITIONNEMENT STERILE A MEMBRANE BI-FONCTIONNELLE**
VORRICHTUNG ZUR ABGABE VON FLÜSSIGKEIT AUS EINER STERILEN VERPACKUNGSFLASCHE MIT BIFUNKTIONELLER MEMBRAN
DEVICE FOR DISPENSING LIQUID FROM A STERILE PACKAGING BOTTLE WITH BIFUNCTIONAL MEMBRANE

(43) Date de publication de la demande: 07.02.2018
(73) Titulaire: Laboratoires THEA, 63100 Clermont-Ferrand (FR)
(72) Inventeur: DEFEMME, Alain, 63400 Chamalières (FR); MERCIER, Fabrice, 63000 Clermont-Ferrand (FR)
(74) Mandataire: Santarelli
(86) Numéro de dépôt international: PCT/IB2015/000423
(87) Numéro de publication internationale: WO 2016/156897

(56) Documents cités:
- WO-A1-2011/095877
- US-A- 5 681 468

## Description

L'invention traite de la technologie des flacons destinés au conditionnement stérile de liquides devant être expulsés hors du flacon par étapes distinctes dans le temps. Elle se rapporte plus précisément aux dispositions qui visent à assurer au mieux le maintien de la stérilité du liquide au fil des étapes de sa consommation, en s'intéressant tant au liquide qui reste dans le réservoir intérieur au flacon qu'à celui qui en est expulsé à chaque étape de la distribution hors du flacon.

L'invention s'applique de façon privilégiée, bien que non restrictive, au domaine des compositions liquides à activité pharmaceutique ou para-pharmaceutique, notamment dans les applications ophtalmologiques. Il s'agit là d'un domaine où les questions de pureté du produit distribué et de précision dans une distribution discontinue, sont particulièrement importantes. Des besoins très proches peuvent aussi se retrouver, par exemple, en cosmétologie ou en dermatologie, notamment pour ce qui concerne la précision dans l'emplacement où amener la goutte ou le jet expulsés à partir d'un réservoir de collecte du liquide interne au flacon, ainsi que, le cas échéant, pour ce qui est de respecter un dosage d'administration prédéterminé à chaque étape de distribution.

Il est connu d'équiper les flacons de conditionnement stérile du type à paroi souple, pour une déformation élastiquement réversible, d'une tête de distribution de liquide annulaire qui s'insère en liaison étanche dans un col rigide du flacon pour en extraire le liquide par l'intermédiaire d'un embout dépassant à l'extérieur du flacon et qui comporte, en travers du conduit interne, une membrane de protection bactériologique capable de faire filtre à l'égard des micro-organismes de la dimension des bactéries et servant ainsi à protéger le liquide contenu dans le flacon des agents polluants qui pourraient y pénétrer depuis le milieu extérieur.

Indépendamment d'une telle protection, on sait que dans les flacons à paroi élastiquement déformable en général, la distribution du liquide est assurée par compression manuelle du flacon, et que le retour du flacon à sa forme d'origine gonflée provoque une entrée d'air venant remplacer le volume de liquide qui en a été extrait. Plutôt que d'équiper le flacon d'une valve mécanique pour gérer l'alternance entre expulsion de liquide et rentrée d'air, il est alors devenu de pratique courante d'utiliser à cet effet des membranes à perméabilité sélective, dans une disposition où elles se laissent alternativement traverser par du liquide et par de l'air en fonction des différentiels de pression de part et d'autre. On connaît, notamment par des documents de brevets, des dispositifs dans lesquels plusieurs membranes sont disposées à l'intérieur du flacon pour réaliser cette perméabilité sélective, tandis que dans des flacons fabriqués industriellement par la Demanderesse, en conformité notamment avec le brevet français publié à l'état de demande sous le numéro FR 2872137 (demande internationale correspondante WO2006 000897), une telle membrane est disposée en travers d'un conduit unique livrant passage aux flux d'air et de liquide entre intérieur et extérieur du flacon, dans un sens et dans l'autre.

WO2011/095877 porte sur un flacon de distribution d'un liquide stérile et a un enseignement similaire.

Ce fonctionnement est rendu possible du fait que l'on utilise comme membrane unique une membrane dite bi-fonctionnelle, qui est réalisée pour partie en un matériau de nature hydrophile et pour partie en un matériau de nature hydrophobe. Le propre de ces membranes est de se laisser traverser tantôt préférentiellement par le liquide aqueux du flacon, tantôt préférentiellement par l'air en provenance de l'extérieur, en fonction d'un différentiel de pression entre ses deux faces, qui varie de manière alternative suivant que la pression fluidique est plus forte ou plus faible du côté interne de la membrane, sur sa face orientée vers l'intérieur du flacon, que la pression s'exerçant sur sa face externe, orientée vers l'extérieur du flacon.

La présence d'une telle membrane, autorisant d'une part le passage du liquide dans le sens de la distribution, sous l'effet d'une pression exercée manuellement par l'utilisateur sur la paroi déformable du flacon, et d'autre part le passage de l'air en sens inverse, de l'extérieur vers l'intérieur du flacon, lorsque la pression sur la paroi est relâchée après la distribution d'une dose de liquide, représente l'un des facteurs qui permettent de conditionner les liquides de pureté biologique dans des flacons multi-doses sans qu'il soit besoin d'incorporer des agents conservateurs dans leur composition.

Les applications usuelles impliquent une distribution de liquide et une aspiration d'air en retour dans la même atmosphère, à savoir le milieu de vie du consommateur, par essence non stérile. Il s'y ajoute que dans nombre d'applications, en ophtalmologie par exemple, il convient de faire particulièrement attention à la contamination par les agents bactériens présents sur la peau ou les muqueuses des personnes en traitement.

D'où le besoin d'améliorer encore la protection antibactérienne, lequel a conduit suivant la présente invention à conférer encore une autre fonction de préservation de la stérilité à la même membrane. US 5 681 468 divulgue un flacon de distribution d'un produit stérile employant une membrane de filtration portant un agent bactériostatique ou bactéricide.

C'est ainsi que l'invention propose de fermer le réservoir de conditionnement stérile du flacon, à la base d'un embout d'expulsion de son contenu hors du flacon, par une membrane à la fois filtrante pour les bactéries ou micro-organismes similaires et semi-perméable bifonctionnelle à l'égard des fluides, qui est en outre chargée dans sa masse d'un agent biocide par effet d'oxydation ionique. Un tel agent est apporté plus particulièrement par des macromolécules porteuses d'ions métalliques à charge positive, telles que celles qu'un art antérieur désormais bien connu propose sous la forme de polymères minéraux de la famille des alumino-silicates, appelés zéolites ou zéolithes, retenant en leur sein des ions métalliques labiles. Parmi les ions utiles, ce sont les ions argent (Ag+ ou Ag++) qui se sont révélés les plus avantageux dans le cadre industriel des membranes de protection anti-bactéries mises en oeuvre suivant la présente invention.

La présente invention consiste donc, au principal, à fournir un flacon de conditionnement stérile fermé par une membrane de protection biologique qui cumule les trois fonctions, celle de membrane filtrante, celle de membrane dite bifonctionnelle séparant les flux de fluides la traversant en distinguant l'air du liquide, celle de matériau chargé d'un agent à effet ionique biocide sur les produits venant à son contact. En cela, elle conduit à mettre à profit les particularités constitutionnelles de la membrane dans un mode de mise en oeuvre de l'effet biocide ionique
se traduisant différemment suivant qu'il s'exerce en présence d'air ou en présence de liquide.

Pour assurer au mieux le rôle d'une telle membrane dans le cadre de sa mise en oeuvre, l'invention prévoit d'ajouter à cette caractéristique principale diverses caractéristiques complémentaires qui viennent combiner leurs effets dans le fonctionnement d'un flacon de conditionnement stérile de liquide du type à paroi élastiquement déformable par pression manuelle. Ces caractéristiques portent principalement sur la réalisation de la membrane elle-même, sur la constitution de la tête de distribution de liquide hors du flacon dans laquelle elle est disposée, en travers d'un large conduit de circulation des fluides, sur la configuration des éléments rigides qui encadrent la membrane en travers de ce conduit. Les caractéristiques de ces trois séries novatrices de l'invention seront d'autant plus efficaces dans la réussite d'une bonne qualité de protection contre les polluants biologiques en toute sécurité dans les conditions normales d'utilisation des flacons qu'elles seront appliquées en combinaison les unes avec les autres.

En ce qui concerne la membrane elle-même, la présente invention prévoit avantageusement qu'elle soit réalisée à partir d'un matériau poreux en matière polymère de nature hydrophile chargée de manière homogène en agent à effet biocide par oxydation ionique, ledit matériau constituant ladite membrane dans toute sa masse et étant ensuite, localement sur une partie de l'étendue de la membrane venant en travers du conduit de circulation des fluides entre intérieur et extérieur du flacon, rendu hydrophobe par un traitement de polymérisation complémentaire préservant son activité biocide.

Ceci permet de ménager un volume approprié pour la mise en contact de la phase gazeuse, constituée par de l'air, avec la matière polymère chargée d'ions actifs au sein de la masse poreuse sur toute l'épaisseur de la membrane. Dans le même sens joue le fait que le matériau de la base hydrophile de la membrane est constitué finement homogène, ce qui exclut les réalisations antérieures des membranes filtres faites d'une matière à base fibreuse retenant entre les fibres des particules chargées. On préfère suivant l'invention partir d'une base polymère fondue comprenant des granules fusibles d'un mélange maître intégrant lui-même des macromolécules minérales porteuses des ions actifs.

Alors que classiquement, la filtration des bactéries demande une porosité fine, ne dépassant pas 0,2 µm, la présence d'un agent biocide au sein de la membrane permet de préserver la stérilité de manière satisfaisante avec des porosités plus grossières, préférentiellement d'environ 0,3 ou 0,4 µm, ou allant plus largement jusqu'à 0,5 µm, ou même jusqu'à 0,6 ou 0,7 µm, voire 1 µm, ce qui est avantageux du point de vue des pertes de charge et permet le traitement de liquides visqueux. En pratique, l'invention prévoit ainsi, suivant un mode de mise en oeuvre préféré, de réaliser la membrane telle qu'elle présente un diamètre de pores moyen adapté à la filtration des micro-organismes de dimensions supérieures à une taille de particules comprise entre 0,3 et 1 µm, en particulier entre 0,3 et 0,6 micromètres.

Les macromolécules support des ions actifs sont avantageusement,, ainsi qu'on l'a déjà indiqué, des polymères minéraux du type des alumino-silicates dans lesquelles les ions actifs sont intégrés, s'agissant plus spécialement, de manière en elle-même connue, d'ions métalliques comme les ions argent ou les métaux similaires sous forme ionique, qui se fixent sur les sites libres des chaînes polysiloxaniques par des liaisons covalentes polaires. Ces polymères minéraux sont de préférence des polymères cristallins. La concentration en ions actifs dans la membrane est de manière préférée, bien que non limitative des conditions d'application de l'invention, choisie comprise entre 100 et 100 000 ppm, en prenant comme exemple le cas d'un polymère minéral à base d'alumino-silicates porteur d'ions argent dans une membrane de porosité d'environ 0,2-0,3 micromètre.

On s'intéressera maintenant aux caractéristiques avantageuses de l'invention concernant le dispositif de distribution de liquide hors d'un flacon de conditionnement stérile suivant l'invention, dans lequel une membrane constituée comme ci-dessus est montée en travers d'un conduit de circulation des fluides entre l'intérieur et l'extérieur du flacon.

On traitera plus particulièrement des moyens d'organisation de la circulation des fluides liquide et air à travers ladite membrane que le dispositif comporte en combinaison avec elle, après avoir rappelé que la membrane bifonctionnelle à cet égard se trouve alors disposée dans le conduit de circulation des fluides entre une partie de large section située dans une nacelle ou insert de raccordement étanche avec le flacon, en amont de la membrane (par rapport au sens du flux liquide sortant), et un canal de section capillaire traversant un embout prolongeant ladite nacelle hors du flacon jusqu'à un orifice d'éjection du liquide. Le long de ce canal, situé en aval de la membrane, les fluides liquide et air circulent en alternance, que ce soit pour sortir du flacon ou pour y rentrer. En effet, dans les applications de ce genre, la membrane en fonctionnement fait office de séparateur de flux en distinguant le liquide et l'air dans leurs mélanges lui parvenant, de par sa nature pour partie hydrophile, donc sélectivement perméable au liquide en présence d'air, et pour partie hydrophobe, donc sélectivement perméable à l'air en présence de liquide.

Parmi les caractéristiques en question, on trouve le fait que dans l'embase d'embout jouxtant la membrane du côté extérieur il est ménagé des moyens de guidage respectifs de l'air aspiré depuis l'extérieur et de tout reliquat de liquide non distribué appelé à rentrer dans le flacon, qui sont configurés, au voisinage de la membrane, de manière à favoriser un effet biocide sur le flux d'air aspiré s'exerçant à l'intérieur de la membrane, au sein de la matière poreuse hydrophobe, et à favoriser plutôt un entraînement de l'agent à effet biocide hors de la membrane par le flux de liquide la traversant en sa partie de nature hydrophile. En exemple préféré de mise en oeuvre de l'invention, ceci s'obtient par des moyens de guidage qui tendent, notamment par des rainures creusées radialement dans la matière de l'embout, à détourner vers la partie de l'étendue de la membrane restée à propriétés hydrophiles tout flux de liquide précédant l'air extérieur aspiré par le canal de l'embout, et qui tendent inversement, grâce éventuellement à des corrugations complémentaires plus superficielles que lesdites rainures, à diviser finement le flux d'air tout en le concentrant sur la partie de l'étendue de la membrane rendue hydrophobe, cette partie hydrophobe étant avantageusement prévue en regard du canal de l'embout à cet effet.

On notera ici que la différentiation de l'effet de l'agent biocide à charge ionique suivant qu'il touche à un flux de liquide ou à un flux d'air, ne saurait s'obtenir d'une membrane réalisée multicouches, même à supposer que parmi une série de couches superposées, en matière poreuse de nature fibreuse ou non, il figurât une couche de nature hydrophobe ou une couche hydrophile, ou encore une couche hydrophobe et une couche hydrophile, dès lors que les zones spécifiquement à propriétés hydrophiles ou hydrophobes de la membrane ne pourraient qu'être inaptes à séparer les flux en distinguant le liquide et l'air dans le flux global lui parvenant.

Suivant encore d'autres caractéristiques de l'invention, les moyens d'organisation de la circulation des fluides dans le dispositif de distribution de liquide hors d'un flacon de conditionnement stérile comportent un tampon poreux monté en travers du conduit de circulation des fluides dans la nacelle ou insert, c'est-à-dire en amont de la membrane, qui est réalisé en un matériau apte à recevoir et absorber l'agent à activité biocide entraîné par le liquide hors de la membrane tout en évitant sa pénétration à l'intérieur du flacon. Sachant que de tels tampons sont connus dans leur fonction de régulateurs de flux par la création d'une perte de charge sur le trajet du liquide poussé hors du flacon, la présente invention prévoit de leur faire jouer un nouveau rôle dans la protection contre les polluants de la stérilité, en les concevant de manière qu'il constituent une réserve d'agent actif en destruction des bactéries.

Dans la mesure où la matière constituant le tampon (" plug " en anglais) n'a pas été chargée d'agent biocide à la fabrication, la réserve d'agent actif disponible ne se constitue qu'à partir de la première utilisation du flacon en consommation de son contenu. Dès lors, en liaison avec les particularités de cette matière, en nature et en porosité notamment, qui jouent sur sa capacité de rétention de l'agent biocide et sur la durée de vie de ce dernier, la longueur axiale du tampon traversée par les fluides en sortie comme en rentrée peut être déterminée, sans difficulté particulière pour les gens du métier, pour que l'agent biodestructeur y soit recueilli progressivement en commençant par les couches du côté extérieur, sans pouvoir aller jusqu'aux dernières couches du côté intérieur. Il s'agit ainsi de ne pas courir le moindre risque de contamination du milieu intérieur au flacon, ce au moins pendant toute la durée normale d'utilisation du flacon jusqu'à consommation complète de son contenu.

On notera qu'un tel tampon poreux n'a pas à jouer les mêmes rôles d'élément filtrant ou de séparateur de flux que la membrane, puisque lui se trouve dans la partie de conduit de circulation des fluides où liquide et air sont libres de se mélanger. Il s'ensuit que dans les formes de mise en oeuvre de l'invention les plus avantageuses en pratique, le tampon est constitué à base de matière fibreuse compactée. Cette matière est de préférence à propriétés hydrophobe, dans la mesure où aussi la porosité est relativement grossière.

Le cas échéant, on peut encore parfaire le résultat de l'activité biocide conjointe de la membrane et du tampon poreux, en complétant la conception du dispositif suivant l'invention par des caractéristiques connues en elles-mêmes qui concernent la constitution de l'embout d'expulsion du liquide extrait du flacon, là en s'intéressant plus spécialement aux applications de l'invention à des flacons à embout compte-gouttes tels que ceux que l'on utilise pour des liquides à distribuer dans une direction vers le bas comme il est de pratique courante en ophtalmologie. C'est ainsi que l'on a recours dans l'invention à des réalisations où l'embout d'expulsion des doses de liquide est constitué en un matériau lui-même chargé d'agent biocide, comme il a été décrit notamment dans la demande internationale de brevets WO 10/013131 (également publiée en demande de brevet US 11/0125111), ce qui permet d'agir sur les fluides déjà à l'extérieur de la membrane, là où les fluides air et liquide circulent en alternance dans un fin canal central de l'embout.

Globalement, la coopération de structures à activité biocide se succédant sur le trajet des fluides apporte une sécurisation de la stérilité telle qu'il est possible d'abaisser sensiblement les exigences de qualité anti-bactéries de la membrane en termes de filtration. Alors qu'il est habituellement prescrit une qualité de filtration s'exprimant par une porosité de 0,1 à 0,2 µm (en diamètre de pores moyen), il devient avantageux, conformément à l'invention, de fixer cette porosité à une valeur comprise entre 0,3 et 1 µm, ou, mieux encore, entre 0,4 et 0,6 ou 0,7 µm, comme on l'a déjà indiqué. La perte de charge subie par le liquide à la traversée de la membrane s'en trouve sensiblement réduite, ce qui peut permettre, quitte à augmenter d'autant la perte de charge à la traversée du tampon régulateur de flux, d'allonger la longueur axiale de ce tampon, et par là d'améliorer son rôle en rétention de charges ioniques disponibles dans leur activité biocide pour un traitement complémentaire des fluides passant dans ses pores. Mais aussi une porosité moins fine de la membrane est en soi fort utile pour ouvrir plus de possibilités dans les applications industrielles des flacons, dans la mesure où leur fonctionnement sera moins sensible à la viscosité du liquide conditionné. Il est ainsi prévu d'utiliser les flacon conçus et construits suivant l'invention pour le conditionnement de collyres à base polymère dont la consistance s'apparente à celle des gels.

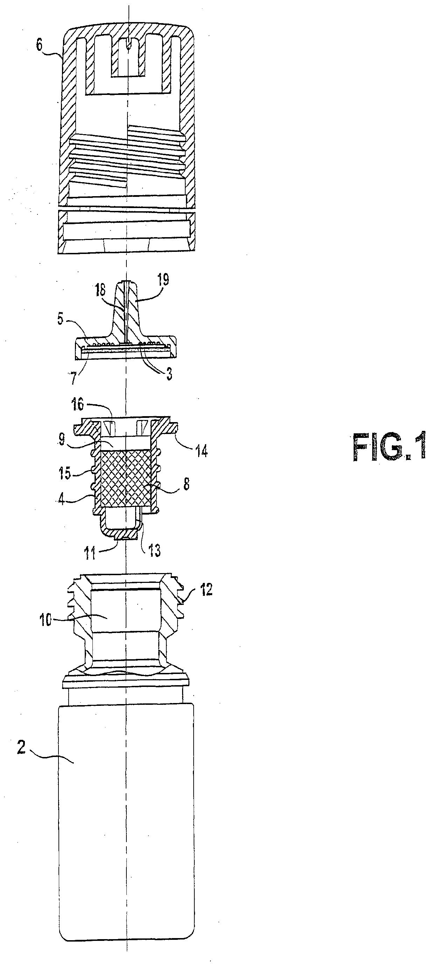

L'invention sera maintenant plus complètement décrite dans le cadre de caractéristiques préférées et de leurs avantages, en se concentrant sur une forme de réalisation particulièrement adaptée au conditionnement d'une solution aqueuse telle qu'un collyre, et en faisant référence aux figures 1 à 5 dans lesquelles :
- la figure 1 représente, en coupe longitudinale et en vue éclatée, les différents éléments d'un flacon de conditionnement stérile à paroi déformable qui est équipé d'un dispositif selon l'invention pour la distribution d'un liquide à expulser du flacon par doses successives à travers une membrane de protection bactériologique, jusqu'à épuisement du liquide qu'il contient à l'état stérile au moment de sa première utilisation ;
- la figure 2 en représente, suivant une coupe longitudinale plus particulièrement le dispositif de distribution, une fois assemblés ses éléments propres pour constituer une tête de distribution de liquide et rentrée d'air extérieur s'insérant dans le col du flacon ;
- la figure 3 illustre, en coupe longitudinale, une variante de réalisation de la tête de distribution de liquide de la figure 2 ;
- la figure 4 illustre la configuration de l'embase de l'embout en sa surface venant en regard de la nacelle ;
- et la figure 5 illustre le fonctionnement de la membrane de protection bactériologique en liaison avec celle du tampon poreux absorbant l'agent biocide sur le trajet de retour des fluides vers l'intérieur du flacon après l'expulsion d'une dose de liquide.

Dans sa constitution générale et tel qu'il est représenté dans tous ses éléments sur la figure 1, le flacon décrit apparaît conforme à la conception usuelle des flacons de conditionnement stérile des brevets antérieurs de la demanderesse. Mais il en diffère par des caractéristiques propres à l'invention qui se répartissent sur ses éléments constitutifs essentiels pour la distribution sous protection bactériologique assurée suivant l'invention, à savoir la membrane (7), le tampon poreux (8) occupant l'espace interne dans la nacelle (4) et l'embase (3) de l'embout (5) de distribution du liquide hors du flacon (2) ainsi que dans leur assemblage relatif lié à la circulation des fluides à travers la membrane.

Conformément à l'invention la membrane que comporte le flacon de conditionnement d'un collyre à l'état stérile est utilisée en séparation des flux de liquide et d'air qui la traversent, en membrane de protection bactériologique par filtration et, par sa matière contenant des macromolécules minérales porteuses d'ions argent à effet biocide d'oxydation ionique, en destruction des bactéries ou micro-organismes analogues dans les fluides qui la traversent.

Une fois montée dans le flacon, en interface entre l'intérieur et l'extérieur du flacon, elle participe à un ensemble d'organisation de la circulation des fluides à travers elle qui conduit à une alternance des flux de liquide et d'air dans le canal d'expulsion à fine section de passage qui est ménagé au centre de la partie effilée en canule (19) de l'embout (5), configuré ici en distributeur de gouttes. La circulation des fluides est alternée en aval de la membrane sur le circuit d'expulsion, alors qu'en amont, l'air et le liquide sont en mélange dans le conduit à large section de passage qui précède la membrane dans la nacelle (4) de raccordement étanche de l'ensemble sur le flacon, dans son col (10).

A l'intérieur de ce conduit, en amont de la membrane du côté intérieur au flacon, on trouve en outre un tampon poreux (8) dont l'une des fonctions reste celle de régulateur de flux qui participe à l'organisation des flux à travers la membrane en créant une perte de charge sur le trajet du liquide sortant du flacon. En fonctionnement, la membrane est en présence d'air ou de liquide indifféremment au cours des opérations de distribution du liquide. Le même tampon a aussi pour rôle, conformément à l'invention, de faire tampon à l'égard du transfert des charges ioniques actives en destruction des bactéries. Il les recueille du liquide qui revient vers l'intérieur du flacon (2) et il les retient dans le matériau poreux, de sorte que l'activité biocide est là disponible pour poursuivre l'action destructrice de bactéries à l'égard aussi de l'air rentrant dans le flacon en compensation de la dose de liquide qui en a été expulsée.

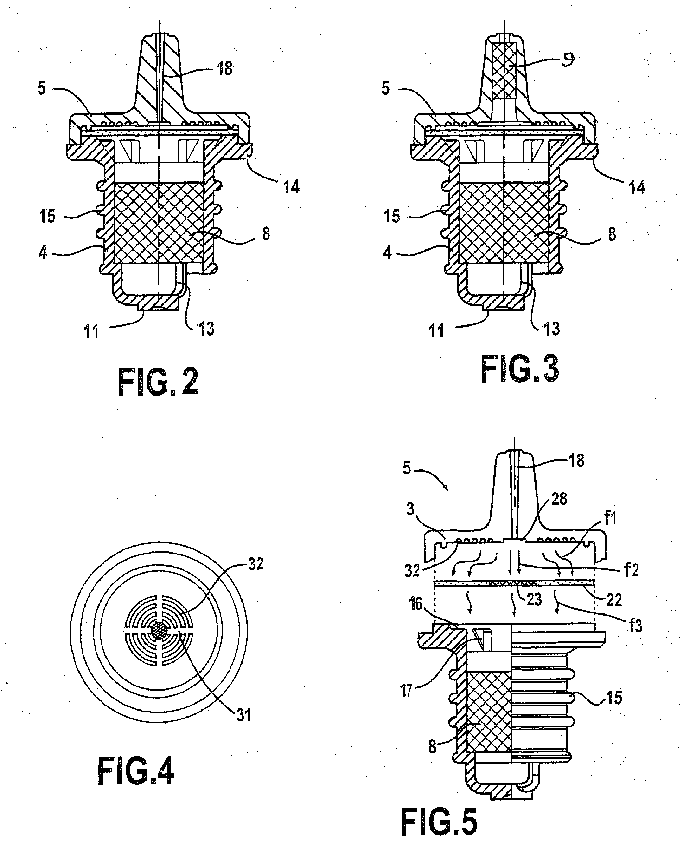

Conformément à l'invention la membrane (7) est constituée de manière homogène dans toute sa masse en ce qui concerne la répartition du polymère cristallin support des ions biocides, plus particulièrement ici une zéolithe à charge d'ions argent. Pour assurer cette homogénéité tant dans la partie de la membrane qui est hydrophile (en 22 sur la figure 5) que dans celle qui est hydrophobe (en 23 sur la figure 5), on a choisi de réaliser la membrane dans une matière polymère à base de résine de polyester modifiée par une résine de polyamide ou de polyéther-sulfone qui est mise en forme à l'état fondu après y avoir incorporé la zéolithe.

Le degré de polymérisation de la matière polymère de base et les caractéristiques de porosité et d'épaisseur sont alors telles qu'elles conduisent à une membrane à propriétés hydrophiles. C'est ensuite que l'on rend hydrophobe la matière, dans sa masse dans toute l'épaisseur de la membrane, mais localement dans une zone définie de l'étendue de sa surface. Cette zone constitue la partie hydrophobe de la membrane alors que le reste de son étendue conserve sa nature hydrophile. Une exposition locale à irradiation sous rayonnement ultra-violet représente un bon moyen de modifier la structure du polymère in situ par des réactions de réticulation radicalaire entre ses constituants en préservant les propriétés des ions actifs emprisonnés dans la zéolithe.

En fonctionnement, lors des opérations de distribution des doses de liquide hors du flacon, la structure de la membrane, en liaison avec l'organisation de la circulation des fluides à travers elle, tend à favoriser une action destructrice des micro-organismes par oxydation ionique qui s'exerce sur le flux d'air au sein même de la matière hydrophobe, par contact entre l'air et le polymère chargé d'ions à la surface des pores, alors qu'au contraire dans la partie hydrophile de la membrane la charge ionique biocide tend à être entraînée par le liquide traversant la membrane.

Le flux d'air entrant par l'embout du flacon est un fluide gazeux qui vient directement en contact avec les ions biocides au sein des pores et qui réagit ainsi avec l'agent à activité biocide immédiatement, alors que le liquide résiduel retournant vers le flacon ne consomme pas sur place la totalité de la charge ionique qu'il est capable d'extraire du matériau de la membrane, de sorte que le flux de liquide retournant vers l'intérieur du flacon entraîne avec lui de la charge ionique qui sera disponible pour jouer plus loin son activité biocide.

Le résultat présente un réel avantage par l'efficacité de la destruction des bactéries assurée sur l'air venant de l'extérieur qui est ainsi traité majoritairement avant de pénétrer dans le flacon. En ce qui concerne la zone hydrophile traversée par le liquide il importe plus que l'effet ionique subsiste dans le liquide ré-aspiré vers l'intérieur du flacon. La charge ionique biocide est alors recueillie et retenue dans la matière poreuse du tampon poreux (8) dans la mesure où les ions biocides ne sont pas consommés dans l'intervalle existant à l'intérieur de la nacelle entre la membrane et le tampon. En travers du conduit de circulation des fluides entre intérieur et extérieur du flacon, en amont de la membrane, les ions y sont alors disponibles pour agir sur tout reliquat de polluant organique y parvenant.

Il est à noter que la longueur axiale du tampon sur le trajet des fluides est choisie suffisante pour que les ions à activité biocide ne soient jamais entraînés à travers lui jusqu'au réservoir intérieur au flacon. En pratique, le choix des caractéristiques dimensionnelles du tampon et de sa porosité, de même que le choix de l'intervalle vide de matière le séparant de la membrane dans la nacelle, résultent d'un compromis entre les besoins de la circulation des fluides en termes de pression et ceux qui sont propres aux effets de la charge ionique initiale de la membrane. Dans le cas particulier décrit le tampon est réalisé en matière polymère à base de polyesters, plus spécialement à base de polyéthylène, qui est en forme à propriétés hydrophiles et à porosité de l'ordre de 100 µm, plus généralement comprise entre 20 et 150 µm en diamètre de pores moyen. Sa longueur suivant l'axe du flacon est comprise entre 3 et 10 mm. Sa distance à la membrane considérée sur sa face intérieure au flacon est du même ordre de grandeur.

De ce côté, en amont de la membrane dans la partie large du conduit de circulation des fluides ménagé par la forme annulaire de la nacelle (4), la surface libre de la membrane est dégagée. Toutefois, des ailettes de soutien (16 et 17 sur la figure 5) sont formées sur la nacelle, du côté interne, pour limiter les contraintes pouvant s'exercer en fonctionnement sur le pourtour de la membrane, là où elle est fixée collée sur une couronne périphérique de l'embase de l'embout d'éjection de liquide, mais elles laissent la membrane libre de se bomber à l'écart de l'embase (3) de l'embout.

En regard de la face externe de la membrane vue en sa nature hydrophile, l'embase (3) de l'embout (5) forme une surface d'appui pour la membrane dans les phases d'expulsion de liquide, qui rejoint la paroi du canal central d'expulsion (18) au niveau de son embouchure évasée (28).

Autour de cette embouchure, la surface libre de l'embout est creusée de rainures radiales offrant une large section de passage au liquide au voisinage de la membrane du côté extérieur au flacon. Ces rainures en disposition rayonnantes sont visibles en 31 sur la vue de face de la figure 4. Leur rôle est de collecter le liquide sortant du flacon en le guidant vers l'embouchure du canal d'expulsion après qu'il ait traversé la membrane en sa zone hydrophile, mais leur rôle est aussi, sur le reliquat de liquide non expulsé qui est réaspiré vers le flacon dans la phase d'entrée d'air en compensation du liquide expulsé, de faciliter que sous la poussée de l'air il soit dirigé vers la zone hydrophile (22, figure 5), en dégageant la zone hydrophobe centrale (23, figure 5) pour l'air qui arrive ensuite dessus.

La surface de l'embase (3) présente par ailleurs des corrugations qui tendent à diviser finement toute veine de circulation d'air prenant naissance au sortir de l'embouchure du canal central de l'embout, ce qui tend à réduire la vitesse à laquelle il traverse ensuite la membrane, alors même que celle-ci est poussée à l'écart de la surface transversale de l'embase de l'embout.

Dans la forme de réalisation préférée d'un embout ainsi réalisé conformément à l'invention, ce notamment dans le cas d'un embout compte gouttes où il importe, pour la qualité de formation de la goutte, que l'air ne se mélange pas au liquide dans le canal d'éjection des gouttes, les corrugations de division de toute veine d'air en circulation sont présentes sous la forme de sillons (32, figure 4) relativement étroits et peu profonds, donc de fine section de passage, qui sont chacun en forme annulaire et répartis en disposition concentrique les uns par rapport aux autres autour du canal central de l'embout. Ces sillons (32) sont creusés en surface de l'embase d'embout, dans les secteurs de l'embase préservés par les rainures (31) de guidage du flux de liquide, là où la surface de l'embase d'embout est plutôt réservée à servir de support d'appui de la membrane lorsque celle-ci est poussée par la pression interne du flacon comprimé pour expulser du liquide.

On comprend qu'en fonctionnement, la configuration particulière de la surface de l'embout venant en regard de la membrane, joue un rôle important dans l'organisation de la circulation des fluides, et ce non seulement en favorisant une alternance entre flux liquide et flux gazeux dans le canal central de l'embout, mais aussi en guidant les fluides sur leur trajet de retour comme le montrent les flèches sur la figure 5. Les flèches f1 illustrent que le reliquat de liquide non expulsé, rappelé en premier, est détourné d'un trajet axial direct et orienté vers la partie hydrophile de la membrane (22). On évite ainsi qu'il soit projeté sur la partie centrale de la membrane, où il tendrait à mouiller la matière de la membrane, de qualité hydrophobe en cet endroit. Le flux d'air aspiré vers le flacon dispose de ce fait d'un libre accès à la matière hydrophile de la membrane en sa partie centrale (23), comme illustré par les flèches f2.

En se référant maintenant aux figures 2 et 3, on insistera sur les modes de mise en oeuvre préférés de l'invention, où la membrane chargée d'ions Ag associée au tampon poreux récepteur des mêmes ions combine en plus ses effets avec ceux d'un embout réalisé lui-même en une matière chargée d'agent biocide, ici également apporté par une charge de zéolithe support des ions.

Une tête de distribution dont l'embout est ainsi chargé d'agent biocide alors que le matériau constituant la nacelle en est dépourvu, est suffisamment décrite dans la demande de brevet antérieure WO2010/013131 de la Demanderesse pour qu'il soit inutile d'en décrire plus les détails ici. Il en est de même pour la variante de la figure 3, sur laquelle on remarque le noyau poreux 9, en matière polymère hydrophobe, qui occupe l'espace interne du canal d'expulsion 18. Ce noyau poreux a pour objet de diviser le flux gazeux en favorisant son contact avec la surface libre intrinsèque sans freiner pour autant exagérément la circulation quand il s'agit d'un flux liquide. Quand la matière constituant le noyau est chargée d'agent biocide, notamment de zéolithe support d'argent ionique, on favorise par là l'effet destructeur de bactéries s'exerçant sur l'air extérieur dès son arrivée dans l'embout.

Quelle que soit la partie de l'embout qui soit chargée d'ions biocide, l'embout lui-même ou un noyau poreux dans le canal central (ou encore d'autres moyens de division du flux comme il a été décrit dans les textes de brevets déjà mentionnés), il est remarquable que dans la tête de distribution complète, l'embout constitue une source de réapprovisionnement de la membrane de protection bactériologique en laissant fuir vers elle, principalement avec le flux liquide, la charge ionique active en destruction des bactéries qui peut en avoir été relarguée sans être entièrement consommée sur place.

Revenant maintenant sur la figure 1, complétée par la figure 2, on observe d'autres détail de réalisation de la tête de distribution de liquide hors d'un flacon de conditionnement stérile qui, pour être en euxmêmes classiques des flacons fabriqués industriellement par la Demanderesse, n'en sont pas moins des moyens participant à la mise en oeuvre de la présente invention par la qualité de la préservation de la stérilité dans le flacon.

En ce sens on notera la présence des godrons périphériques externes (15) de la nacelle (4), qui assurent une étanchéité contre les bactéries avec le col du flacon (10) au niveau du tampon poreux (8). On notera aussi la configuration du bouchon capuchon (6) qui est telle que, quand il est vissé (en 12) sur le col du flacon, il vient obturer l'embouchure externe du canal 18. Il a entre autres pour rôle d'assurer une chute de pression en aval de la membrane qui évite que celle-ci soit mouillée par le liquide contenu dans le flacon tant que la bague d'inviolabilité (26) n'a pas été fracturée pour une première utilisation (première expulsion d'une goutte de collyre).

Dans le même ordre d'idée, on notera encore la forme de la nacelle (8) à son extrémité amont, à l'intérieur du flacon. Son utilité se fera en premier lieu sentir dans des réalisations destinées à la distribution de collyres à particularités physico-chimiques tensio-actives ou visqueuses, et dans de tels cas les moyens illustrés seront avantageusement exploités en combinaison avec des formes de mise en oeuvre plus spécifiques de l'invention, à savoir celles prévoyant une membrane de porosité relativement grossière conduisant à une moindre qualité de protection par filtration des micro-organismes en même temps qu'un tampon poreux réalisé particulièrement efficace du point de vue de la rétention de la charge ionique encore active en destruction des bactéries. Ces moyens résident dans la configuration formant des arches (13) autour d'une pastille centrale (11) et se disposant dans le flacon (2) au-delà de son col (10). Ils ont été amplement décrits dans la demande de brevet WO 2011/095877. Ils contribuent à une organisation de la circulation des fluides favorable aux besoins de la présente invention dans le cas des mêmes liquides.

Par des essais venant compléter les données de calculs préliminaires, on déterminera au cas par cas les spécifications à respecter pour les différents éléments intervenant dans la migration des ions actifs (en termes notamment de dimensions, porosité, composition du matériau, concentration initiale en charge ionique labile), en fonction de la composition du liquide à expulser hors du flacon, de ses caractéristiques physico-chimiques, du degré de stérilité à préserver, en fonction aussi des conditions d'utilisation du flacon et de sa durée de vie utile.

Dans la pratique, pendant toute la durée de vie utile du flacon suivant sa première utilisation, le tampon poreux interne à la nacelle fait obstruction aux ions Ag venant de la membrane 5 et à chaque étape de distribution du liquide par expulsion d'une goutte de collyre il s'imprègne progressivement de l'activité ionique non consommée, de proche en proche dans les couches que le liquide traverse. L'agent biocide y est ainsi disponible, pour agir tant sur le liquide que sur l'air au sein de la matière poreuse, et détruire ainsi les derniers polluants organiques éventuellement encore présents avant qu'ils ne pénètrent dans la réserve de liquide que l'on souhaite conserver stérile jusqu'à consommation complète du liquide.

Dans le même temps, puisque l'on part d'un flacon dont la tête de distribution de liquide est pourvue d'un embout en polymère incorporant des particules enfermant des ions argent, la membrane se recharge en ions argent à l'occasion des différentes opérations de distribution d'une dose de liquide, quand à chaque fois elle se laisse traverser par un excès de liquide non expulsé qui revient dans le flacon après avoir léché le matériau de l'embout. On a en effet observé que, là aussi, le flux gazeux de l'air extérieur pénétrant dans le flacon consomme les ions à charge positive quand il est au contact du matériau, tandis qu'en extrayant la charge ionique active du matériau, le flux en phase liquide ré-aspiré tend plus à l'entraîner avec lui qu'à la consommer immédiatement sur place. Comme la membrane reste imbibée de liquide d'une opération de distribution à l'autre, l'activité ionique s'y poursuit grâce à cette réalimentation répétitive qui recharge la membrane en ions biocides.

A titre d'exemple, on a utilisé un flacon réalisé conformément à l'invention telle qu'elle vient d'être décrite pour conditionner des collyres et l'on a effectué différents essais au cours de la durée normale d'utilisation du flacon, pour une consommation simulée d'une goutte chaque matin et chaque soir, chaque dose de distribution étant estimée à 30 microlitres pour un flacon contenant 10 millilitres du collyre initialement.

Les mesures ont été répétées pour plusieurs solutions de collyres, en déterminant à chaque fois la teneur en argent du liquide restant disponible dans le flacon d'abord à l'ouverture , du flacon (première utilisation), puis après un mois d'utilisation du flacon, puis aussi après deux mois d'utilisation. Avec des résultats se chiffrant toujours à moins de 0,1 µg / ml, il s'avère que les valeurs observées restent bien inférieures aux exigences de la pratique pour un collyre ne contenant pas de conservateur antibactérien. Il n'y a aucun risque d'agressivité pour les muqueuses de l'œil.

Dans des essais tout différents, on a mesuré la concentration en ions argent existant au sein du tampon poreux présent dans la partie nacelle du dispositif suivant l'invention, après avoir découpé le tampon en trois tranches de disque une fois le collyre consommé, en simulation comme précédemment à une goutte matin et soir chaque jour. On a clairement observé que d'une part la teneur en argent dans la matière solide du tampon décroît le long du tampon, dans sa direction axiale, des couches externes par où pénètrent les fluides au retour d'une phase de distribution aux couches internes plus proches de la réserve de liquide contenue dans le flacon, et que d'autre part elle diminue dans chaque couche au cours du temps, ce qui laisse entendre que l'argent est consommé à chaque passage des fluides à travers le flacon.

Les essais ci-dessus ont été réalisés en utilisant un flacon équipé d'une tête de distribution de liquide dans laquelle, conformément à l'invention, la charge ionique initiale dans la membrane, exprimée en poids d'élément Ag+ ou Ag++ , était choisie comprise entre 100 et 10 000 ppm pour une membrane de 2,3 cm2 d'étendue globale. Bien entendu il s'agit de cas d'exemple, que l'on pourra adapter en modifiant les données chiffrées en fonction des conditions rencontrées en pratique dans chaque cas d'application de l'invention.

## Revendications

1. Dispositif de distribution de liquide hors d'un flacon stérile du type comportant une membrane de protection bactériologique (7) montée en travers d'un conduit de circulation des fluides entre l'intérieur et l'extérieur du flacon pour retenir les micro-organismes éventuellement présents à l'extérieur, et constituée en un matériau réalisé en une matière à propriétés hydrophiles dans une première partie (22) de l'étendue de la membrane en travers dudit conduit et à propriétés hydrophobes dans une seconde partie (23) de cette étendue,
le dispositif comportant des moyens d'organisation de la circulation des fluides liquide et air à travers ladite membrane qui comportent un tampon poreux (8) monté en travers du conduit de circulation des fluides en amont de la membrane du côté intérieur au flacon,
le dispositif comportant en outre en aval de la membrane du côté extérieur au flacon un embout de distribution de liquide hors du flacon par doses successives,
**caractérisé en ce que** ladite membrane est constituée en un matériau intégrant un agent à activité biocide par oxydation ionique,
**en ce que** le tampon poreux (8) est réalisé en un matériau de nature et de porosité telles qu'il est apte à recevoir et absorber ledit agent à activité biocide entraîné par le liquide hors de la membrane tout en évitant sa pénétration à l'intérieur du flacon,
et **en ce que** l'embout est réalisé en une matière polymère non poreuse chargée d'un agent biocide de même type que celui présent dans ladite membrane et apte à migrer à travers le matériau vers sa surface libre pour en être progressivement relargué au passage des fluides, de sorte que l'excédent non consommé au contact du matériau est entraîné avec tout flux liquide retournant vers le flacon, en servant ainsi à recharger la membrane en agent biocide.

2. Dispositif suivant la revendication 1, **caractérisé en ce que** ledit agent à activité biocide est à base d'ions métalliques à charge positive.

3. Dispositif suivant la revendication 2, **caractérisé en ce que** ledit agent à activité biocide est à base d'ions argent supportés par une structure macromoléculaire minérale, en particulier de type zéolite.

4. Dispositif suivant l'une des revendications 1 à 3, **caractérisé en ce que** ladite membrane est réalisée à partir d'un matériau poreux en matière polymère de nature hydrophile chargée d'agent biocide par oxydation ionique la constituant dans toute sa masse, ladite matière polymère étant ensuite, localement dans toute l'épaisseur de la membrane sur une partie de son étendue venant en travers dudit conduit, rendue hydrophobe par un traitement de polymérisation complémentaire préservant son activité biocide.

5. Dispositif suivant la revendication 4, **caractérisé en ce que** ladite matière polymère initialement de nature hydrophile est rendue localement hydrophobe par une réaction de réticulation radicalaire entre ses constituants qui est initiée par irradiation locale de la membrane sous rayonnement ultraviolet.

6. Dispositif suivant la revendication 5, **caractérisé en ce que** la partie hydrophobe de la membrane est située en sa zone centrale.

7. Dispositif suivant l'une quelconque des revendications précédentes, **caractérisé en ce que** lesdits moyens d'organisation de la circulation des fluides comportent en outre, du côté extérieur de la membrane, des moyens de guidage respectifs de l'air aspiré depuis l'extérieur et de tout reliquat de liquide non distribué appelé à rentrer dans le flacon, qui sont configurés de manière à favoriser un effet biocide sur le flux d'air aspiré s'exerçant à l'intérieur de la membrane et à favoriser un entraînement de l'agent à effet biocide hors de la membrane par le flux de liquide la traversant.

8. Dispositif suivant l'une des revendications 4 à 6, dans lequel ladite membrane (7) se monte entre une nacelle (4) de raccordement étanche avec le flacon et un embout (5) prolongeant ladite nacelle hors du flacon, et dans lequel ledit conduit de circulation des fluides se limite dans ledit embout à un canal central (18) de section réduite pour une circulation alternée des fluides liquide et air, **caractérisé en ce que** lesdits moyens d'organisation de la circulation des fluides comportent en outre, du côté extérieur de la membrane, des moyens de guidage respectifs de l'air aspiré depuis l'extérieur et de tout reliquat de liquide non distribué appelé à rentrer dans le flacon, qui sont configurés de manière à détourner vers la partie de l'étendue de la membrane à propriétés hydrophiles tout flux de liquide précédant l'air extérieur dans ledit canal et à faciliter l'impact du flux d'air sur la partie de l'étendue de la membrane à propriétés hydrophobes, cette partie hydrophobe de la membrane étant avantageusement prévue en regard dudit canal à cet effet.

9. Dispositif suivant la revendication 8, **caractérisé en ce que** lesdits moyens de guidage sont ménagés en surface d'une embase dudit embout en regard de la membrane et **en ce qu'**ils comportent des rainures radiales de large section en disposition rayonnante autour de l'embouchure dudit canal et des sillons circulaires concentriques de fine section autour de ladite embouchure en regard de la partie hydrophobe de la membrane.

10. Dispositif suivant l'une quelconque des revendications précédentes, **caractérisé en ce que** la membrane présente un diamètre de pores moyen adapté à la filtration des micro-organismes de dimensions supérieures à une taille de particules comprise entre 0,3 et 1 µm, en particulier entre 0,3 et 0,6 micromètres.

11. Dispositif selon l'une quelconque des revendications précédentes, **caractérisée en ce que** le tampon poreux (8) est réalisé de manière à jouer en outre un rôle de régulateur de flux contribuant à l'organisation de la circulation des fluides à travers ladite membrane.

12. Dispositif suivant la revendication précédente, dans lequel ledit tampon est réalisé en une matière hydrophobe fibreuse et en ce qu'il présente un diamètre de pores moyen équivalent compris entre 20 et 120 micromètres.

13. Dispositif selon l'une des revendications précédentes, dans lequel le matériau constituant ledit embout est à base de polymère de type polyéthylène.

14. Dispositif selon l'une des revendications précédentes dans lequel le matériau constituant ledit embout est à base de polymère de type polyéthylène et contient dans sa masse un agent à effet biocide par oxydation ionique sous forme d'ions Ag intégrés dans des macromolécules minérales.

15. Dispositif selon l'une quelconque des revendications précédentes, dans lequel ledit embout est configuré en embout compte-gouttes, avec un canal central de section capillaire débouchant à l'extérieur du flacon par un orifice de calibrage de goutte.

16. Dispositif suivant la revendication 15, **caractérisé en ce que** le canal central d'expulsion comporte un noyau de division du flux y circulant, avantageusement réalisé sous forme de noyau poreux (9), qui est lui-même constitué en un matériau intégrant un agent biocide, sous la forme d'ions argent sur support de macromolécules minérales.

17. Dispositif suivant l'une quelconque des revendication présentes, dans son application à la réalisation d'un flacon de distribution de liquide par doses successives à paroi à déformation élastiquement réversible par pression manuelle, assurant l'aspiration d'air extérieur lors d'un retour de la paroi à sa forme d'origine après expulsion d'une dose de liquide à travers la membrane sous l'effet d'une pression manuelle exercée sur ladite paroi.

## Patentansprüche

1. Vorrichtung zur Abgabe von Flüssigkeit aus einem sterilen Flakon vom Typ, der eine bakteriologische Schutzmembran (7) enthält, die quer zu einer Fluidzirkulationsleitung zwischen dem Innenbereich und dem Außenbereich des Flakons montiert ist, um Mikroorganismen zurückzuhalten, die eventuell im Außenbereich vorhanden sind, und aus einem Material bestehend, das aus einem Stoff mit hydrophilen Eigenschaften in einem ersten Teil (22) der Membranausdehnung quer zur Leitung hergestellt ist und mit hydrophoben Eigenschaften in einem zweiten Teil (23) dieser Ausdehnung, wobei die Vorrichtung Mittel zur Organisation der Zirkulation von Fluiden, Flüssigkeit und Luft durch die Membran enthält, die einen porösen Pfropfen (8) enthalten, der quer zur Fluidzirkulationsleitung stromaufwärts der Membran an der Innenseite des Flakons montiert ist,
wobei die Vorrichtung ferner stromabwärts von der Membran auf der Außenseite des Flakons einen Stutzen zur Abgabe von Flüssigkeit aus dem Flakon in aufeinanderfolgenden Dosen enthält,
**dadurch gekennzeichnet, dass** die Membran aus einem Material besteht, das eine Substanz mit biozider Aktivität durch ionische Oxidation beinhaltet,
dass der poröse Pfropfen (8) aus einem Material mit einer solchen Beschaffenheit und Porosität hergestellt ist, dass er in der Lage ist, die Substanz mit biozider Aktivität, die durch die Flüssigkeit aus der Membran mitgerissen wird, aufzunehmen und zu absorbieren, während ihr Eindringen in den Innenbereich des Flakons vermieden wird,
und dass der Stutzen aus einem nicht porösen polymeren Stoff hergestellt ist, der mit einer bioziden Substanz der gleichen Art wie die in der Membran vorhandenen beladen ist, und in der Lage ist, durch das Material hindurch zu seiner freien Oberfläche zu wandern, um beim Durchgang der Fluide allmählich von ihr befreit zu werden, so dass der Überschuss, der bei Kontakt mit dem Material nicht verbraucht wird, mit jedem Flüssigkeitsstrom, der in den Flakon zurückkehrt, mitgerissen wird, wodurch die Membran mit biozider Substanz nachgeladen wird.

2. Vorrichtung nach Anspruch 1, **dadurch gekennzeichnet, dass** die Substanz mit biozider Aktivität auf positiv geladenen Metallionen basiert.

3. Vorrichtung nach Anspruch 2, **dadurch gekennzeichnet, dass** die Substanz mit biozider Aktivität auf Silberionen basiert, die von einer mineralischen makromolekularen Struktur, insbesondere vom Typ Zeolith, getragen werden.

4. Vorrichtung nach einem der Ansprüche 1 bis 3, **dadurch gekennzeichnet, dass** die Membran ausgehend von einem porösen Material aus einem polymerem Stoff hydrophiler Beschaffenheit hergestellt ist, der durch ionische Oxidation mit einer bioziden Substanz beladen ist, die ihn in ihrer gesamten Masse ausmacht, wobei der polymere Stoff dann lokal in der gesamten Dicke der Membran über einen Teil ihrer Ausdehnung, der quer zur Leitung steht, hydrophob gemacht wird durch eine ergänzende Polymerisationsbehandlung, die seine biozide Aktivität bewahrt.

5. Vorrichtung nach Anspruch 4, **dadurch gekennzeichnet, dass** der polymere Stoff mit der ursprünglichen hydrophilen Beschaffenheit durch eine radikalische Vernetzungsreaktion zwischen seinen Komponenten, die durch lokale Bestrahlung der Membran unter ultravioletter Strahlung initiiert werden, lokal hydrophob gemacht wird.

6. Vorrichtung nach Anspruch 5, **dadurch gekennzeichnet, dass** sich der hydrophobe Teil der Membran in ihrem zentralen Bereich befindet.

7. Vorrichtung nach einem der vorhergehenden Ansprüche, **dadurch gekennzeichnet, dass** die Mittel zur Organisation der Zirkulation der Fluide ferner, auf der Außenseite der Membran, entsprechende Mittel enthalten zur Führung der vom Außenbereich angesaugten Luft und aller verbleibenden nicht abgegebenen Flüssigkeiten, die bestimmt sind, um in den Flakon zurückzugelangen, und so konfiguriert sind, dass sie eine biozide Wirkung auf den angesaugten Luftstrom fördern, der im Innenbereich der Membran ausgeübt wird, und ein Mitreißen der Substanz mit biozider Wirkung aus der Membran heraus durch den sie durchquerenden Flüssigkeitsstrom fördern.

8. Vorrichtung nach einem der Ansprüche 4 bis 6, wobei die Membran (7) zwischen einem mit dem Flakon dicht verschließenden Kranz (4) und einem Stutzen (5), der den Kranz aus dem Flakon heraus verlängert, montiert wird, und wobei sich der Fluidzirkulationsleitung im Stutzen an einem zentralen Kanal (18) mit reduziertem Querschnitt für eine abwechselnde Zirkulation von Fluiden, Flüssigkeit und Luft beschränkt, **dadurch gekennzeichnet, dass** die Mittel zur Organisation der Zirkulation der Fluide ferner, auf der Außenseite der Membran, die entsprechenden Mittel enthalten zur Führung der vom Außenbereich angesaugten Luft und aller verbleibenden nicht abgegebenen Flüssigkeiten, die bestimmt sind, um in den Flakon zurückzugelangen, und so konfiguriert sind, dass sie alle Flüssigkeitsströme in den Teil der Ausdehnung der Membran mit hydrophilen Eigenschaften umleiten, der Außenluft im Kanal vorausgehend, und den Einfluss des Luftstroms auf den Teil der Ausdehnung der Membran mit hydrophoben Eigenschaften erleichtern, wobei dieser hydrophobe Teil der Membran zu diesem Zweck vorteilhafterweise gegenüber dem Kanal vorgesehen ist.

9. Vorrichtung nach Anspruch 8, **dadurch gekennzeichnet, dass** die Führungsmittel an der Oberfläche eines Unterteils des Stutzens gegenüber der Membran angeordnet sind, und dass sie radiale Rillen mit breitem Querschnitt in Strahlenanordnung um die Mündung des Kanals enthalten und konzentrische kreisförmige Furchen mit dünnem Querschnitt um die Mündung gegenüber dem hydrophoben Teil der Membran.

10. Vorrichtung nach einem der vorhergehenden Ansprüche, **dadurch gekennzeichnet, dass** die Membran einen mittleren Porendurchmesser aufweist, der zur Filtration von Mikroorganismen mit Abmessungen geeignet ist, die größer sind als eine Partikelgröße zwischen 0,3 und 1 um, insbesondere zwischen 0,3 und 0,6 Mikrometer.

11. Vorrichtung nach einem der vorhergehenden Ansprüche, **dadurch gekennzeichnet, dass** der poröse Pfropfen (8) so hergestellt ist, dass er ferner eine Rolle als Strömungsregler spielt, der zur Organisation der Zirkulation von Fluiden durch die Membran hindurch beiträgt.

12. Vorrichtung nach dem vorhergehenden Anspruch, wobei der Pfropfen aus einem faserigen hydrophoben Stoff hergestellt ist und einen äquivalenten mittleren Porendurchmesser zwischen 20 und 120 Mikrometern aufweist.

13. Vorrichtung nach einem der vorhergehenden Ansprüche, wobei das Material, aus dem der Stutzen besteht, auf einem Polymer vom Typ Polyethylen basiert.

14. Vorrichtung nach einem der vorhergehenden Ansprüche, wobei das Material, aus dem der Stutzen besteht, auf Polymer vom Typ Polyethylen basiert und in seiner Masse eine Substanz mit biozider Wirkung durch ionische Oxidation in Form von Ag-Ionen, die in mineralischen Makromoleküle beinhaltet sind, umfasst.

15. Vorrichtung nach einem der vorhergehenden Ansprüche, wobei der Stutzen als Pipettenstutzen konfiguriert ist mit einem zentralen Kanal mit Kapillarquerschnitt, der im Außenbereich des Flakons in einer Öffnung mit Tropfenkalibrierung ausmündet.

16. Vorrichtung nach Anspruch 15, **dadurch gekennzeichnet, dass** der zentrale Ausleitungskanal einen Kern zur Aufteilung des darin zirkulierenden Stroms enthält, der vorteilhafterweise in Form eines porösen Kerns (9) hergestellt ist, der selbst aus einem Material besteht, das eine biozide Substanz beinhaltet, in Form von Silberionen mit dem Träger von mineralischen Makromolekülen.

17. Vorrichtung nach einem der vorhandenen Ansprüche, in ihrer Anwendung bei der Herstellung eines Flakons zur Abgabe von Flüssigkeit in aufeinanderfolgenden Dosen mit elastisch reversibler Wandverformung durch manuellen Druck, die das Ansaugen von Außenluft bei einer Rückkehr der Wand in ihre ursprüngliche Form gewährleistet, nachdem eine Dosis von Flüssigkeit durch die Membran hindurch ausgeleitet wurde durch manuellen Druck, der auf die Wand ausgeübt wurde.

## Claims

1. A device for dispensing liquid out of a sterile bottle of the type including a bacteriological protection membrane (7) mounted across a conduit for circulating the fluids between the inside and the outside of the bottle to retain the micro-organisms which are possibly present outside, and made of a material with hydrophilic properties in a first portion (22) of the extent of the membrane across said conduit and with hydrophobic properties in a second portion (23) of this extent,
the device including means for organising the circulation of liquid and air fluids through said membrane which include a porous pad (8) mounted across the fluid circulation conduit upstream of the membrane on the inner side of the bottle,
the device further including, downstream of the membrane on the outer side of the bottle, a tip for dispensing liquid out of the bottle in successive doses,
**characterised in that** said membrane is made of a material integrating an agent with biocidal activity by ionic oxidation,
**in that** the porous pad (8) is made of a material of such nature and porosity that it is capable of receiving and absorbing said agent with biocidal activity driven by the liquid out of the membrane while avoiding its penetration inside the bottle,
and **in that** the tip is made of a non-porous polymer material loaded with a biocidal agent of the same type as that present in said membrane and capable of migrating through the material towards the free surface thereof to be gradually released therefrom as fluids pass, such that the excess which is not consumed in contact with the material is driven with any liquid flow returning to the bottle, thus used to recharge the membrane with biocidal agent.

2. The device according to claim 1, **characterised in that** said agent with biocidal activity is based on positively charged metal ions.

3. The device according to claim 2, **characterised in that** said agent with biocidal activity is based on silver ions supported by a mineral macromolecular structure, in particular of the zeolite type.

4. The device according to one of claims 1 to 3, **characterised in that** said membrane is made from a porous polymer material of hydrophilic nature loaded with biocidal agent by ionic oxidation constituting it in its entire mass, said polymer material then being, locally throughout the thickness of the membrane over a portion of its extent coming across said conduit, made hydrophobic by a complementary polymerisation treatment preserving its biocidal activity.

5. The device according to claim 4, **characterised in that** said polymer material initially of hydrophilic nature is made locally hydrophobic by a radical crosslinking reaction between the constituents thereof which is initiated by local irradiation of the membrane under ultraviolet radiation.

6. The device according to claim 5, **characterised in that** the hydrophobic portion of the membrane is located in the central area thereof.

7. The device according to any one of the preceding claims, **characterised in that** said means for organising the circulation of fluids further include, on the outer side of the membrane, respective means of guiding the air sucked from the outside and any remaining undispensed liquid intended to enter the bottle, which are configured so as to promote a biocidal effect on the sucked air flow being exerted inside the membrane and to promote drive of the agent with biocidal effect outside the membrane by the liquid flow passing therethrough.

8. The device according to one of claims 4 to 6, wherein said membrane (7) is mounted between a nacelle (4) for sealed connection with the bottle and a tip (5) extending said nacelle outside the bottle, and wherein said fluid circulation conduit is limited in said tip to a central channel (18) of reduced section for an alternating circulation of the liquid and air fluids, **characterised in that** said means for organising the circulation of fluids further include, on the outer side of the membrane, respective means for guiding the air sucked from the outside and any remaining undispensed liquid intended to enter the bottle, which are configured so as to divert, towards the portion of the extent of the membrane with hydrophilic properties, any liquid flow preceding the outside air in said channel and to facilitate the impact of the air flow on the portion of the extent of the membrane with hydrophobic properties, this hydrophobic portion of the membrane being advantageously provided facing said channel for this purpose.

9. The device according to claim 8, **characterised in that** said guide means are formed on the surface of a base of said tip facing the membrane and **in that** they include radial slots of large section in a radiating arrangement around the mouthpiece of said channel and concentric circular grooves of fine section around said mouthpiece facing the hydrophobic portion of the membrane.

10. The device according to any one of the preceding claims, **characterised in that** the membrane has an average pore diameter adapted to the filtration of microorganisms of dimensions greater than a particle size comprised between 0.3 and 1 um, in particularly between 0.3 and 0.6 micrometres.

11. The device according to any one of the preceding claims, **characterised in that** the porous pad (8) is made so as to further act as a flow regulator contributing to the organisation of the circulation of fluids through said membrane.

12. The device according to the preceding claim, wherein said pad is made of a fibrous hydrophobic material and in that it has an equivalent average pore diameter of comprised between 20 and 120 micrometres.

13. The device according to one of the preceding claims, wherein the material constituting said tip is based on a polyethylene type polymer.

14. The device according to one of the preceding claims, wherein the material constituting said tip is based on a polyethylene type polymer and contains in its mass an agent with biocidal effect by ionic oxidation in the form of Ag ions integrated into mineral macromolecules.

15. The device according to any one of the preceding claims, wherein said tip is configured as a dropper tip, with a central channel of capillary section opening outside the bottle through a drop calibration orifice.

16. The device according to claim 15, **characterised in that** the central expulsion channel includes a core for dividing the flow circulating therein, advantageously produced in the form of a porous core (9), which itself consists of a material integrating a biocidal agent, in the form of silver ions supported by mineral macromolecules.

17. The device according to any one of the present claims, in the application thereof to the production of a bottle for dispensing liquid in successive doses with a wall with an elastically reversible deformation by manual pressure, ensuring the suction of external air during a return of the wall to its original shape after expulsion of a dose of liquid through the membrane under the effect of a manual pressure exerted on said wall.
